**Europäisches Patentamt**

**European Patent Office**

**Office européen des brevets**

(19)

(11) Numéro de publication: **0 237 379
B1**

(12) **FASCICULE DE BREVET EUROPEEN**

(45) Date de publication du fascicule du brevet:
06.09.89

(51) Int. Cl.⁴: **A61B 17/58**

(21) Numéro de dépôt: 87400265.2

(22) Date de dépôt: 05.02.87

(54) Cheville d'ancrage bio-compatible et prothèse comportant une telle cheville.

(30) Priorité: 11.02.86 FR 8601840

(43) Date de publication de la demande:
16.09.87 Bulletin 87/38

(45) Mention de la délivrance du brevet:
06.09.89 Bulletin 89/36

(84) Etats contractants désignés:
CH DE FR GB IT LI NL

(56) Documents cités:
EP-A- 0 040 300
GB-A- 2 084 468

(73) Titulaire: SOCIETE EUROPEENNE DE PROPULSION,
24 rue Salomon de Rothschild, F-92150 Suresnes(FR)

(72) Inventeur: Vives, Michel, No. 33 Le Bois de la Landotte,
Le Taillan§F-33320 Eysines(FR)
Inventeur: Buttazzoni, Bernard, 23 Boulevard Auguste
Cleussa, Le Galion F-13007 Marseille(FR)

(74) Mandataire: Clanet, Denis et al, Cabinet Beau de
Loménie 55, rue d'Amsterdam, F-75008 Paris(FR)

ACTORUM AG

## Description

La présente invention concerne une cheville d'ancrage bio-compatible permettant la fixation d'une plaque de support ou autre prothèse sur un corps osseux tel qu'une paroi corticale d'un os.

Dans le domaine de la chirurgie osseuse, le problème de la bio-compatibilité des prothèses n'a été résolu jusqu'à présent que partiellement, car si les nouvelles prothèses et plaques en matériaux composites du type carbone-carbone sont bio-compatibles, l'ancrage actuel de ces prothèses et plaques est réalisé par des vis spéciales en titane ou en alliage chrome-cobalt-molybdène dont les filets s'engagent dans la partie osseuse entourant les trous de fixation percés à cet effet dans la paroi osseuse.

On pouvait imaginer de remplacer les vis métalliques par des vis composites du type carbone-carbone pour rendre bio-compatible l'ensemble des éléments de support et de fixation et pour supprimer ainsi définitivement les risques de rejet des prothèses, dus aux alliages métalliques.

Cependant, la conception connue de ces vis spéciales de grande longueur et comportant un filetage très effilé à faible diamètre en fond de filet ne permet pas l'emploi de matériaux composites pour des dimensions de vis identiques ou similaires. En effet, la faible section transversale de ces vis ne supporterait pas les couples de torsion que l'on y applique lors du montage desdites vis.

L'un des buts de la présente invention est de proposer une cheville d'ancrage bio-compatible dont la structure permet l'emploi de matériaux moins résistants que les alliages métalliques.

Il est évident que les filets des vis d'ancrage connues pénètrent, soit par vissage, dans les parties osseuses entourant les trous taraudés de fixation, soit par autotaraudage, en s'y taillant progressivement leur chemin et que ce type de fixation exige une matière osseuse encore relativement solide.

Un autre but de l'invention est de proposer une cheville d'ancrage qui évite toute entaille pratiquée dans la paroi des trous de fixation et permet d'établir des contacts dont les forces sont sensiblement perpendiculaires à l'axe et à la paroi cylindrique des trous de fixation, tout en créant une force de retenue axiale.

Encore un autre but de l'invention est de proposer une cheville dont la face cylindrique de contact s'applique, le cas échéant, de façon élastique contre la paroi interne du trou de fixation correspondant.

L'invention a également pour but de proposer une cheville d'ancrage dont le verrouillage proprement dit ne s'effectue qu'après que l'une des extrémités de la cheville ait traversé le trou de fixation et dépasse de celui-ci.

Ces différents buts de l'invention sont au moins partiellement atteints du fait que la cheville d'ancrage réalisée en une matière bio-compatible avec le tissu humain, tel que du carbone-carbone, comprend deux demi-chevilles présentant chacune une demi-tête d'appui et une tige d'ancrage de pourtour semi-cylindrique et symétrique l'un par rapport à l'autre, et un plan de séparation radial passant au moins partiellement par l'axe de la cheville et se prolongeant jusqu'à la face intérieure de la demi-tête à laquelle est raccordée la tige d'ancrage correspondante, la tige ayant une longueur suffisante pour pouvoir dépasser d'une paroi osseuse d'une distance au moins égale au diamètre du trou de fixation, ainsi que des moyens de blocage au voisinage de l'extrémité libre de la cheville susceptibles d'écarter l'une de l'autre les deux demi-chevilles pour les appliquer élastiquement sur au moins une partie de leur longueur contre la paroi intérieure du trou de fixation.

Grâce à cette conception, on peut réaliser des prothèses totalement bio-compatibles ayant un ancrage suffisamment solide et aisé à mettre en place et adapté aux caractéristiques mécaniques des matériaux composites tels que le carbone-carbone.

D'autres caractéristiques et avantages de l'invention ressortiront de la description suivante de plusieurs modes de réalisation ainsi que des revendications annexés.

La description des différents modes de réalisation est faite en référence aux dessins annexés sur lesquels :

- les figures 1 et 2 représentent respectivement en coupe longitudinale et en coupe transversale un os tubulaire muni d'une prothèse fixée sur l'os à l'aide de vis filetées connues ;
la figure 3 montre en coupe longitudinale un os tubulaire munie d'une plaque ou prothèse fixée sur l'os à l'aide d'un premier mode de réalisation (partie droite de la figure) et d'un deuxième mode de réalisation (partie gauche de la figure) d'une cheville d'ancrage conforme à l'invention ;
- la figure 4 montre en coupe transversale selon la ligne IV-IV de la figure 3, un ensemble constitué par une paroi corticale d'un os tubulaire, une plaque de support appliquée contre la face extérieure dudit os et le premier mode de réalisation de la cheville d'ancrage selon l'invention ;
- les figures 5a et 5b sont des vues en perspective des deux demi-chevilles constituant le premier mode de réalisation de la cheville d'ancrage selon l'invention ;
- la figure 6 montre en coupe transversale un détail de la partie supérieure d'une cheville d'ancrage ;
- la figure 7 montre en coupe transversale selon la ligne VII-VII de la figure 3, un ensemble constitué par deux parties de paroi corticale opposées d'un os tubulaire, une plaque de support appliquée contre la face extérieure dudit os et un deuxième mode de réalisation de la cheville d'ancrage conforme à l'invention ;
la figure 8 est une vue en perspective d'une demi-cheville du deuxième mode de réalisation de la cheville ;

la figure 9 est une vue en élévation à travers la partie moyenne et inférieure du deuxième mode de réalisation de la cheville d'ancrage ; et

- la figure 10 est une vue en plan de plusieurs coupes horizontales axialement écartées de la cheville d'ancrage selon la figure 9, et du trou de fixation de l'os recevant la partie inférieure de ladite cheville.

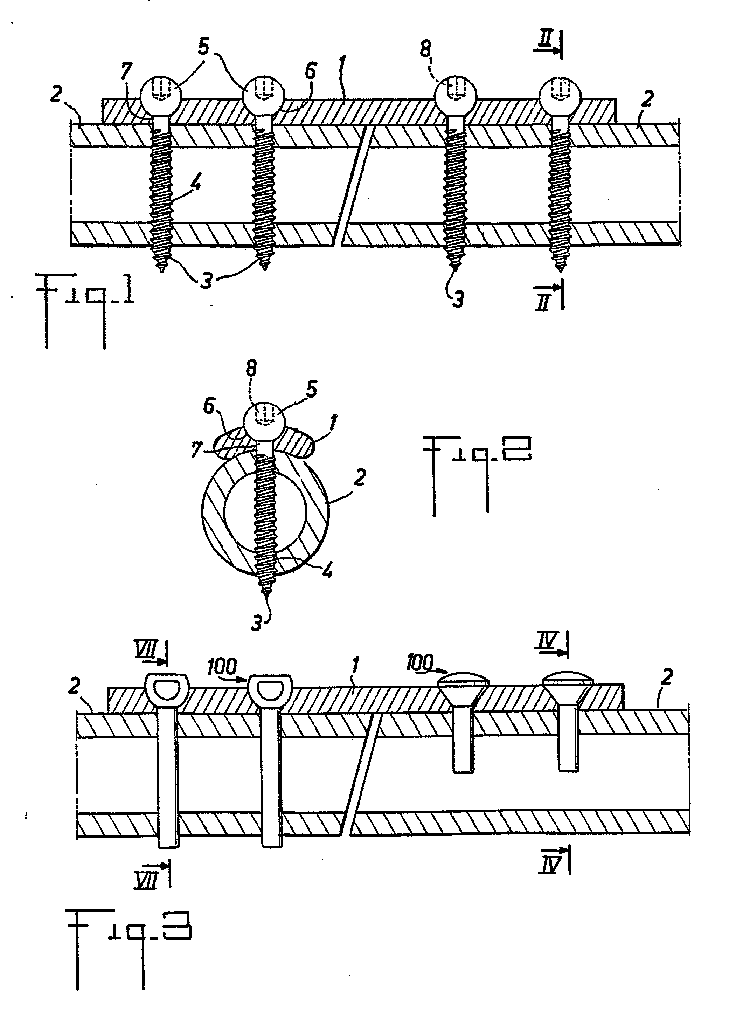

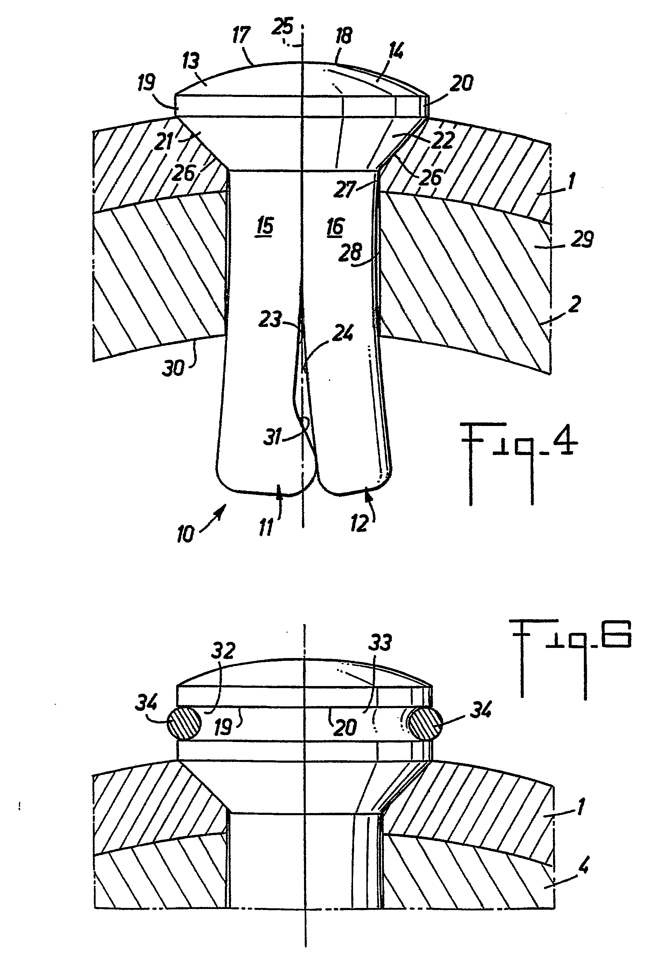

Comme on peut le voir sur les figures 1 et 2, une plaque de prothèse 1, par exemple en carbone-carbone, de forme adaptée à celle de l'os tubulaire 2 y est fixée à l'aide de plusieurs vis métalliques 3 dont les filets 4 de chacune s'engagent dans la paroi de deux trous de fixation alignés et préalablement percés dans l'os tubulaire 2. La tête de vis 5 présente généralement un élargissement sphérique ou tronconique dont la partie inférieure est noyée dans des logements sphériques ou tronconiques correspondants 6 de la plaque 1, prolongés, chacun, par un trou de passage 7. La partie supérieure de ces têtes de vis 5 présente généralement un évidement à six pans 8 destiné à recevoir une clé pour la rotation des vis 3.

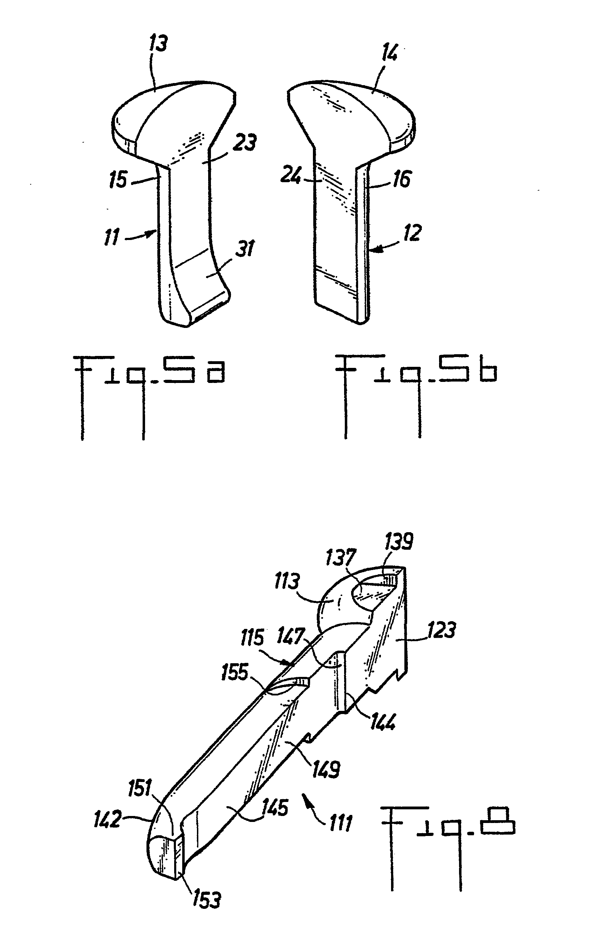

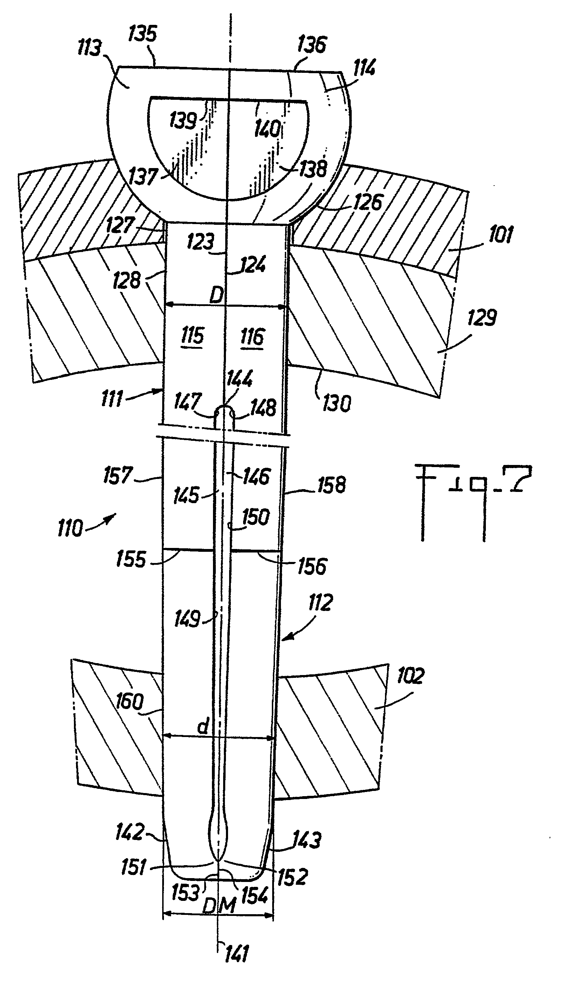

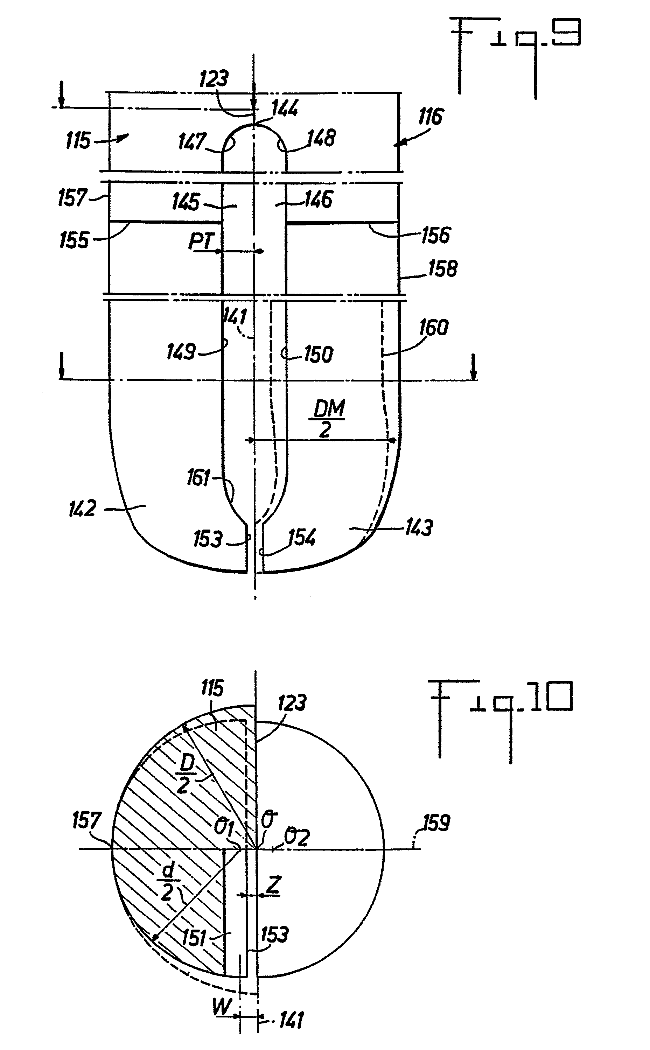

La cheville d'ancrage 10 telle que représentée sur les figures 3 à 10 des dessins est réalisée en un matériau bio-compatible comme la plaque de prothèse 1, par exemple en carbone-carbone.

Selon un premier mode de réalisation, la cheville d'ancrage 10 comprend deux demi-chevilles 11, 12 comportant, chacune, à une des extrémités, une demi-tête d'appui 13, 14 et une tige d'ancrage 15, 16 raccordée à ladite tête 13, 14. Selon le mode de réalisation représentée sur les figures 4 à 6, chaque tête d'appui 13, 14 présente, en direction de la tige 15, 16, une calotte semi-sphérique 17, 18, un tronçon semi-cylindrique 19, 20 et une partie semi-tronconique 21, 22 se rétrécissant en direction de la tige d'ancrage 15, 16 dont la section transversale se confond, à l'endroit de la jonction avec ladite tête, avec celle de la petite base de ladite partie semi-tronconique 21, 22.

Chaque demi-cheville 11, 12 présente une face plane verticale de contact 23, 24 qui s'étend dans un plan radial 25 passant par l'axe de la cheville 10, lorsque les deux demi-chevilles sont appliquées l'une contre l'autre le long de leurs faces de contact correspondantes 23, 24. La partie semi-tronconique 21, 22 de chaque demi-cheville 11, 12 est noyée dans un logement tronconique correspondant 26 de la plaque 1, ce logement 26 étant prolongé par un trou de passage cylindrique 27 qui se trouve en alignement avec un trou de fixation 28 percé dans la paroi corticale 29 de l'os tubulaire 2 et présentant un diamètre intérieur dont la valeur est égale à celle du diamètre intérieur du trou de passage 27 et légèrement supérieure à celle du diamètre extérieur des deux demi-chevilles assemblées 11, 12 au niveau du trou de passage 27 de la plaque de prothèse 1.

La longueur de la tige d'ancrage 15, 16 de chacune des deux demi-chevilles 11, 12 est sensiblement la même et est telle que l'extrémité libre de la tige dépasse de la face intérieure 30 de la paroi corticale 29 d'une distance au moins égale au diamètre du trou de fixation 28.

Selon le premier mode de réalisation représenté sur les figures 3 à 6, la première demi-cheville 11 (voir figures 4 et 5a) comporte, près de son extrémité libre, une rampe de blocage 31 qui s'étend à partir de la face verticale de contact 23, sous un angle relativement faible par rapport à la verticale, par exemple 10 à 15°, vers le bas et vers la face de contact verticale 24 (voir figures 4 et 5b) de l'autre demi-cheville 12. Il est à noter que la face de contact plane verticale 24 de la deuxième demi-cheville s'étend de son extrémité supérieure, c'est-à-dire de la demi-tête d'appui 14, jusqu'à son extrémité libre dite inférieure. Cette rampe de blocage 31 qui, en coopération avec la partie inférieure de la face de contact 24 de la deuxième demi-cheville 12, constitue les moyens de blocage de la cheville 10, prend naissance de préférence à une certaine distance de la paroi corticale 29, par exemple à une distance de l'ordre de 1 à 1,5 fois le rayon du pourtour semi-cylindrique de la tige d'ancrage 15.

L'ancrage de la plaque de prothèse 1 sur l'os tubulaire 2 s'effectue de la manière suivante :

Avant la pose de la plaque 1 sur l'os 2, on perce, à l'aide d'un gabarit de perçage, dans la paroi corticale 29 dudit os, un trou de fixation 28 en alignement avec le trou de passage 27 de ladite plaque 1 et de même diamètre que ce dernier. Il est à rappeler qu'à l'exception de la partie munie de la rampe de blocage 31, les deux tiges d'ancrage semi-cylindriques 15, 16 sont symétriques et présentent le même diamètre que celui du trou de fixation 28 ou un diamètre légèrement inférieur à ce dernier.

On met d'abord en place la première demi-cheville 11 munie de la rampe de blocage 31, de telle sorte que la partie tronconique 21 de sa demi-tête d'appui 13 repose dans son logement correspondant 26. Ensuite, on met en place la deuxième demi-cheville 12 dont la face de contact 24 glisse d'abord le long de la face de contact 23 de la première demi-cheville 11 jusqu'à ce que son extrémité rencontre la rampe de blocage 31. En forçant la deuxième demi-cheville 12 à avancer jusqu'à ce que sa demi-tête d'appui 14 repose également dans le logement 26 de la plaque 1, par exemple à l'aide d'une bouterolle ou d'un marteau, les extrémités libres des deux demi-chevilles 11, 12 dépassant du trou de fixation, vont s'écarter progressivement l'une de l'autre en fléchissant élastiquement et en s'appliquant contre la paroi du trou de fixation. L'effort de serrage se dirigeant perpendiculairement à l'axe général de la cheville 10, n'affecte pas, en général, la résistance mécanique de l'os en raison de la flexion des deux demi-chevilles et de l'emplacement du point de blocage à l'extérieur de la paroi corticale. Par contre, la force de friction dans le sens axial et surtout l'effet de coin agissant entre le trou de fixation de la paroi corticale 26 et l'extrémité libre élargie de la cheville 10 interdit tout déplacement axial d'une demi-cheville par rapport à l'autre et par rapport au trou de fixation, de sorte qu'est assuré un bon ancrage de la plaque de prothèse 1 sur l'os 2 et est empêché tout retrait de la cheville 10 sans destruction de celle-ci.

On notera que le fléchissement élastique obtenu avec les demi-chevilles conformément à l'invention est nettement plus important que s'il s'était agi de demi-chevilles métalliques. En effet, des demi-chevilles métalliques atteindraient rapidement le seuil de déformation plastique et les efforts d'application contre la paroi du trou de fixation diminueraient rapidement en fonction des infimes déplacements que les demi-chevilles subissent dans le temps.

Par contre, grâce au matériau carbone-carbone qui procure un fléchissement élastique plus important (effet ressort), les efforts d'application contre la paroi du trou persistent dans le temps malgré les infimes déplacements des demi-chevilles.

En outre, cette importance du fléchissement élastique permet un ajustement relativement précis des efforts d'applications en agissant sur les dimensions des éléments en présence, notamment le diamètre du trou de fixation.

Pour empêcher tout retrait d'une demi-cheville 11, 12 sous l'effet des sollicitations mécaniques et vibratoires, on peut prévoir d'autres moyens d'adhésion d'une demi-cheville à l'autre. On peut, par exemple, enduire la face de contact 23 ou 24 au niveau de la tête d'appui 13, 14 d'une colle bio-compatible ou bien munir la partie semi-cylindrique 19, 20 d'une gorge annulaire 32, 33 et y loger un jonc torique fendu 34, par exemple également réalisé en un matériau bio-compatible tel que le carbone-carbone.

L'ancrage de la cheville 10 est d'ailleurs rapidement consolidé par une colonisation osseuse autour de l'extrémité libre de la cheville 10 et dans l'intervalle formé entre les parties écartées des faces de contact 23, 24 des demi-chevilles 11, 12.

Il a été expérimentalement observé que cette colonisation osseuse apporte des avantages à deux niveaux différents :
- en premier lieu, dans l'intervalle entre les deux demi-chevilles où elle réduit considérablement le risque de glissement axial relatif des demi-chevilles l'une par rapport à l'autre ;
- en second lieu, autour des extrémités libres dépassant du trou de fixation où elle aboutit à former un bloc qui entoure ces extrémités libres et fait corps avec elles en empêchant pratiquement tout retrait de la cheville hors du trou.

La cheville d'ancrage 110 représentée aux figures 7 à 10 et correspondant à un deuxième mode de réalisation est plus particulièrement, mais non exclusivement utilisée lorsqu'elle doit traverser deux parties de paroi corticale opposées d'unmême os tubulaire. Ce deuxième mode de réalisation de la cheville d'ancrage 110 comprend également deux demi-chevilles 111, 112 avec les demi-têtes d'appui correspondantes 113, 114 dont la forme est cette fois-ci presque entièrement semi-sphérique à l'exception d'une face supérieure d'appui plane et horizontale 135, 136 perpendiculaire à l'axe de la cheville 110, et de deux faces verticales parallèles planes opposées 137, 138 surmontées par un épaulement extérieur 139, 140. Les demi-chevilles 111, 112 présentent, dans ce cas particulier, une forme tout fait symétrique par rapport à un plan de symétrie 141 contenant ledit axe. Chaque demi-cheville 111, 112 comprend en dessous de la demi-tête d'appui correspondante 113, 114, une tige d'ancrage 115, 116 qui comporte un pourtour extérieur de forme générale semi-cylindrique s'effilant légèrement vers son extrémité libre de façon à présenter une extrémité libre de forme semi-tronconique 142, 143.

La partie supérieure de chaque demi-cheville 111, 112 possède une face de contact 123, 124 qui se confond avec le plan de symétrie 141, lorsque ces deux demi-chevilles 111, 112 sont assemblées en une cheville unique 110. Ces deux faces de contact 123, 124, dont chacune se situe dans un plan radial de chaque demi-cheville 111, 112, s'étendent sur une partie seulement de la hauteur totale de la cheville 110 et de préférence, sur une hauteur supérieure à l'épaisseur de la plaque de prothèse 101 et de la paroi corticale adjacente 129, mais inférieure à la somme résultant de l'épaisseur de la plaque 101 et du rayon de l'os tubulaire correspondant 102. D'une façon générale, la hauteur de la face de contact 123, 124 est telle que son bord inférieur 144 dépasse de la face intérieure 130 de la paroi corticale 129 adjacente à la plaque 101 d'une distance de l'ordre du rayon du pourtour semi-cylindrique de la tige d'ancrage 115, 116.

A partir de ce bord inférieur 144 de la face de contact 123, 124, chaque demi-cheville 111, 112 comprend un évidement 145, 146 de faible profondeur transversale PT de l'ordre de quelques dixièmes de millimètres, cet évidement étant délimité en haut, par une dépouille 147, 148 se raccordant au bord inférieur 144 de la face de contact 123, 124, par un fond plan 149, 150 parallèle à la face de contact 123, 124 et au plan de symétrie 141, ainsi qu'à l'extrémité libre 142, 143 de la tige d'ancrage 115, 116, par une rampe de verrouillage 151, 152 s'étendant parallèlement au bord inférieur 144 de la face de contact 123, 124 et présentant une face d'appui 153, 154 parallèle à la face de contact supérieure 123, 124 et au fond plan 149, 150 de l'évidement 145, 146.

Comme on peut le voir sur la figure 7, la longueur de la cheville 110, et notamment des tiges d'ancrage 115, 116, est suffisante pour que, après la mise en place de la cheville 110 sur la plaque 101 et l'os tubulaire 102, l'extrémité libre 142, 143 des tiges 115, 116 dépasse de l'autre côté de l'os opposé à celui en contact avec la plaquette 101, d'une distance à peu près de l'ordre du diamètre de la cheville 110 au niveau des tiges assemblées.

Sur son pourtour extérieur, chaque tige d'ancrage 115, 116 est subdivisée en deux parties par un épaulement horizontal 155, 156 qui s'effile progressivement à partir du fond plan 149, 150 jusqu'à la génératrice 157, 158 située dans le plan radial passant par l'axe de la cheville 110 et perpendiculaire au plan de symétrie 141, aux faces de contact 123, 124 et aux fonds 149, 150 des évidements 145, 146. Cet épaulement horizontal 155, 156 se situe à peu près entre le premier tiers supérieur et le tiers médian de l'évidement 145, 146.

La partie supérieure de chaque tige d'ancrage 115, 116, située au-dessus de l'épaulement médian horizontal 155, 156 présente un pourtour semi-cylindrique dont la section transversale est un demi-cercle dont le diamètre D a son origine O sur l'axe général et dans le plan de symétrie 141 de la cheville d'ancrage 110, la section transversale de la cheville 110 étant à ce niveau un cercle de diamètre D.

La partie inférieure de chaque tige d'ancrage 115, 116, située au-dessous de l'épaulement médian 155, 156 comporte aussi un pourtour semi-cylindrique dont l'axe est situé dans un plan radial 159 perpendiculaire au plan de symétrie 141 de la cheville 110 et passant par l'axe général (0) de celle-ci, de sorte que la section transversale de la partie inférieure de chaque tige d'ancrage présente également une forme en demi-cercle dont le diamètre d est inférieur au diamètre D, et dont l'origine O1 ou O2 est située dans le plan radial 159 de part et d'autre du plan de symétrie 141 à une distance W de celui-ci.

Il convient de rappeler que, dans le cas du deuxième mode de réalisation, la plaque 101 comporte un logement semi-sphérique 126 destiné à recevoir la partie inférieure des demi-têtes d'appui 113, 114 des demi-chevilles 111, 112, ce logement étant raccordé au trou de passage cylindrique 127 pour les tiges d'ancrage 115, 116. Le diamètre du trou de passage 127 est légèrement supérieur au diamètre D du premier trou de passage 128 pratiqué dans la partie de paroi corticale adjacente à la plaque de prothèse 101, ce diamètre D étant également celui du pourtour cylindrique de la partie supérieure de la cheville 110. A l'opposé du premier trou de passage 128, la paroi corticale 129 de l'os tubulaire 102 est munie d'un deuxième trou de passage 160 qui est coaxial au premier (128) et qui présente un diamètre intérieur d égal au diamètre extérieur d du pourtour cylindrique de la partie inférieure de chaque tige d'ancrage 115, 116.

La valeur de décalage latéral W des centres O1 et O2 des demi-cercles des sections transversales de la partie inférieure des tiges d'ancrage 15, 16 est déterminée par la relation

$$W = \frac{D - d}{2}$$

D étant le diamètre du premier trou de passage 128 et du demi-cercle de la section transversale de la partie supérieure des tiges d'ancrage semi-cylindriques 115, 116, et d étant le diamètre du demi-cercle de la section transversale de la partie inférieure des tiges d'ancrage 115, 116, de même que du deuxième trou de passage 160.

Selon un exemple numérique, on peut choisir D = 4,5 mm et d = 4,0 mm.

Cette disposition particulière a pour conséquence que seule la génératrice 157 ou 158 située dans le plan radial 159 perpendiculaire au plan de symétrie 141 est une droite continue appartenant aussi bien à la partie supérieure qu'inférieure des tiges d'ancrage 115, 116, tandis que toutes les autres génératrices du pourtour semi-cylindrique de ces tiges 115, 116 sont interrompues au niveau de l'épaulement transversal 155, 156.

Comme précédemment mentionné, la partie inférieure de l'évidement 145, 146 est délimitée par une rampe de verrouillage 151, 152 qui se raccorde en continu au fond 149, 150 de l'évidement 145, 146 et dont la face d'appui verticale 153, 154 se trouve à une distance Z du plan de symétrie 141, lorsque les deux demi-chevilles 111, 112 sont assemblées sans contrainte, de telle sorte que leurs faces de contact 123, 124 reposent l'une contre l'autre. La valeur de Z est, de préférence, de l'ordre de 3W/5 et celle de PT de l'ordre de 2W.

Dans ces circonstances, les deux faces d'appui 153, 154 des rampes de verrouillage 151, 152 sont écartées l'une de l'autre d'une distance égale à 2Z ou 6W/5, lorsque les deux faces de contact 123, 124 reposent l'une contre l'autre et qu'aucune autre contrainte n'agit sur les deux demi-chevilles assemblées 111, 112.

Dans ce cas, les deux génératrices 157, 158 sont écartées l'une de l'autre, même dans la partie inférieure de la cheville d'une distance égale à D.

Lors de la fixation d'une plaque de prothèse 101 sur l'os 102, et après le perçage des deux trous de fixation 128, 160 coaxiaux, mais de diamètres différents D, d, on met en place la première demi-cheville 111 dans le trou de passage 127 et les deux trous de fixation 128, 160. Dans ce cas, la partie inférieure de la demi-cheville 111 va être déviée en direction du plan de symétrie 141, car la paroi du deuxième trou de fixation est radialement décalée de

$$\frac{D - d}{2}$$

vers l'axe général de la cheville 110 par rapport à celle du premier trou de fixation 128 et la génératrice 157 doit être placée dans une position légèrement oblique par rapport à la verticale pour permettre à la tige d'ancrage 115 de passer travers le deuxième trou de fixation 160. Lorsque la première demi-tête d'appui 113 repose dans son logement 126 de la plaque 101, on met en place la deuxième demi-cheville 112 dont l'extrémité inférieure 143 ne rencontre point d'obstacle du fait que sa rampe de verrouillage 152 peut se déplacer sans difficulté dans l'évidement 145 de la première demi-cheville 111 jusqu'à ce qu'elle rencontre le flanc incliné supérieur 161 de la rampe de verrouillage 151 de la première demi-cheville 111.

En descendant le long de ce flanc 161 incliné de haut en bas de l'extérieur vers le plan de symétrie 141, l'extrémité inférieure 143 de la deuxième demi-cheville 112 oblige l'extrémité 142 de l'autre demi-cheville à s'écarter comme elle, élastiquement vers l'extérieur en des sens opposés jusqu'à ce que la face d'appui 154 de la deuxième demi-cheville 112 s'immobilise devant la face d'appui 153 de la première demi-cheville 111 et y reste fortement et élastiquement appliquée. Dans ce cas, les deux tiges d'ancrage 115, 116 fléchissent légèrement et élastiquement d'une part, vers l'intérieur en direction de l'évidement 146 ou 145 de l'autre tige 116, 115 au niveau du deuxième trou de fixation 160, et d'autre part, vers l'extérieur de façon à former des coins en dessous dudit deuxième trou 160.

En effet, le diamètre d du deuxième trou de fixation est inférieur à l'écartement D des deux génératrices 157, 158 et à la dimension maximale DM de la section transversale de l'extrémité inférieure de la cheville 110 dépassant du deuxième trou 160, cette dimension maximale DM prise dans le plan radial 159 étant déterminée par la relation suivante :

$$DM = 2 \left(\frac{d}{2} + W - Z\right) \text{ ou } DM = d + \frac{4}{5} W \text{ pour } Z = \frac{3}{5} W.$$

Si l'on prend, dans un exemple numérique D = 4,5 mm, d = 4,00 mm, W = 0,25 mm, Z = 0,15 mm, on obtient pour DM = 4,2 mm, ce qui veut dire que l'effet de coin appliqué par les deux tiges d'ancrage 115, 116 à la paroi intérieure du deuxième trou de fixation 160 est dû à un écartement de 0,2 mm des faces cylindriques des extrémités inférieures des deux tiges 115, 116. On remarquera également qu'au niveau du deuxième trou de fixation 160, le fond 149, 150 de l'évidement 145, 146 doit se déformer élastiquement vers l'intérieur d'une valeur égale à W pour permettre au pourtour cylindrique des tiges 115, 116 de s'adapter au diamètre d du deuxième trou 160.

Comme précédemment mentionné, l'ancrage de la cheville 110 est rapidement consolidé par une colonisation osseuse autour de la cheville 110 de part et d'autre de la paroi corticale 102 et dans l'espace formé par les deux évidements 145, 146 des deux demi-chevilles 111, 112.

Le fait que le point de contact ou de verrouillage de la cheville 110 se trouve (à l'endroit des rampes 151, 152) en dehors de la paroi corticale 102 et est accessible de l'extérieur rend un éventuel démontage de la cheville 110 plus aisé que dans le cas des systèmes connus.

Bien entendu, on peut encore réaliser d'autres variantes mettant en oeuvre l'effet de coin et de blocage mutuel de deux demi-chevilles sans sortir du cadre de la présente invention défini par les revendications annexées.

En outre, l'invention vise également toutes les plaques de prothèse bio-compatible susceptibles d'être fixées sur l'os à l'aide de la cheville bio-compatible conforme à l'invention.

**Revendications**

1. Cheville d'ancrage (10, 110) permettant la fixation d'une plaque de support ou autre prothèse (1, 101), sur un corps osseux tel qu'une paroi corticale (29, 129) d'un os (2, 102), caractérisée en ce qu'elle est réalisée en un matériau bio-compatible avec le tissu humain et comprend deux demi-chevilles (11, 12 ou 111, 112) présentant chacune une demi-tête d'appui (13, 14 ou 113, 114) et une tige d'ancrage (15, 16 ou 115, 116) de pourtour semi-cylindrique et, le cas échéant, symétrique l'une par rapport à l'autre, et un plan de séparation radial (41, 141) passant au moins partiellement par l'axe de la cheville (10 ou 110), et se prolongeant jusqu'à la face intérieure de la demi-tête (13, 14 ou 113, 114) à laquelle est raccordée la tige d'ancrage correspondant, la tige (15, 16 ou 115, 116) ayant une longueur suffisante pour pouvoir dépasser d'une paroi osseuse (29 ou 129) d'une distance au moins égale au diamètre du trou de fixation (28, 160), ainsi que des moyens de blocage (31; 151,152) au voisinage de l'extrémité libre de la cheville susceptibles d'écarter l'une de l'autre les deux demi-chevilles (11, 12 ou 111, 112) pour les appliquer élastiquement sur au moins une partie de leur longueur contre la paroi intérieure du trou de fixation (28; 160).

2. Cheville d'ancrage selon la revendication 1, caractérisée en ce que chaque demi-cheville (11, 12 ou 111, 112) présente au moins dans sa partie supérieure une face de contact plane (23, 24 ou 123, 124) par laquelle elle repose contre l'autre demi-cheville (12, 11 ou 112, 111).

3. Cheville d'ancrage selon l'une des revendications 1 et 2, caractérisée en ce que la première demi-cheville (11) comporte, près de son extrémité libre, une rampe de blocage (31) qui s'étend à partir de la face verticale de contact (23) sous un angle relativement faible vers le bas et vers la face de contact (24) de l'autre demi-cheville (12), avec laquelle elle coopère en vue d'un écartement mutuel des extrémités inférieures des deux demi-chevilles lorsque celles-ci sont engagées dans le trou de fixation (28) d'une paroi osseuse (29) et en vue de la production d'un effet de coin vis-à-vis de la paroi dudit trou de fixation (28).

4. Cheville d'ancrage selon l'une des revendications 1 et 2, caractérisée en ce que les deux demi-chevilles (111, 112) présentent une longueur suffisante pour traverser les trous coaxiaux (128, 160) de diamètres différents (D, d) pratiqués dans un os tubulaire (102) et dépasser de celui-ci par leurs extrémités libres d'une distance de l'ordre du diamètre de la cheville assemblée (110), que les faces de contact (123, 124) s'étendent à la partie supérieure des demi-chevilles (111, 112) sur une hauteur supérieure à l'épaisseur de la plaque de prothèse (101) et de la paroi corticale (129) adjacente de l'os tubulaire, qu'à partir du bord inférieur (144) de la face de contact (123, 124), chaque demi-cheville (111, 112) comprend un évidement (145, 146) de faible profondeur (PT) délimité en haut par une dépouille (147, 148) se raccordant au bord inférieur (144), par un fond plan (149, 150) parallèle la face de contact (123, 124) et au plan de symétrie (141), ainsi qu'à l'extrémité libre de chaque tige d'ancrage (115), (116), par une rampe de verrouillage (151, 152) comportant une face d'appui (153, 154) et servant à l'écartement mutuel des extrémités libres des tiges d'ancrage, et que sur son pourtour, chaque tige d'ancrage (115, 116) est subdivisée en deux parties semi-cylindriques par un épaulement horizontal (155, 156) qui s'effile progressivement à partir du fond plan (149, 150) de l'évidement (145, 146) jusqu'à la génératrice (157, 158) située dans le plan radial (159) perpendiculaire au plan de symétrie (141).

5. Cheville d'ancrage selon l'une des revendications 1 à 4, caractérisée en ce que le point de contact et de verrouillage entre les deux demi-chevilles (11, 12 ; 111, 112) est situé en dehors de la paroi corticale (29; 129) de l'os (2;102).

6. Ensemble de prothèse et de chevilles bio-compatibles utilisant des chevilles d'ancrage selon l'une des revendications 1 à 5.

## Patentansprüche

1. Fixierungsnagel (10, 110) zur Befestigung einer Trägerplatte oder einer anderen Prothese (1, 101) an einem Knochenkörper wie etwa der Corticalwand (29, 129) eines Knochens (2, 102), dadurch gekennzeichnet, daß er aus einem mit dem menschlichen Gewebe biokompatiblen Material hergestellt ist und zwei Halbnägel (11, 12 oder 111, 112), die jeweils einen Haltehalbkopf (13, 14 oder 113, 114) und einen Fixierungsstift (15, 16 oder 115, 116) von halbzylindrischem Umfang bilden und gegebenenfalls in bezug aufeinander symmetrisch sind, eine radiale Trennfläche (41, 141), die wenigstens teilweise die Achse des Nagels (10 oder 110) schneidet und sich bis zur Innenseite des Halbkopfes (13, 14 oder 113, 114), an den der entsprechende Fixierungsstift anschließt, erstreckt, wobei der Stift (15, 16 oder 115, 116) eine hinreichende Länge besitzt, so daß er aus der Knochenwand (29 oder 129) in einer Länge hervorsteht, die mindestens gleich dem Durchmesser des Befestigungsloches (28, 160) ist, und in der Nähe des freien Endes des Nagels eine Blokkiereinrichtung (31; 151, 152), die dazu geeignet ist, die beiden Halbnägel (11, 12 oder 111, 112) gegeneinander aufzuspreizen, um sie elastisch wenigstens auf einem Teil ihrer Länge gegen die Innenwand des Befestigungsloches (28; 160) zu drücken, aufweist.

2. Fixierungsnagel gemäß Anspruch 1, dadurch gekennzeichnet, daß jeder Halbnagel (11, 12 oder 111, 112) wenigstens in seinem oberen Teil eine ebene Verbindungsfläche (23, 24 oder 123, 124) aufweist, mit der er sich dem anderen Halbnagel (12, 11 oder 112, 111) aufstützt.

3. Fixierungsnagel gemäß einem der Ansprüche 1 und 2, dadurch gekennzeichnet, daß der erste Halbnagel (11) nahe seinem freien Ende eine Blockierrampe (31) aufweist, die sich ausgehend von der vertikalen Verbindungsfläche (23) unter einem relativ kleinen Winkel nach unten und zur Verbindungsfläche (24) des anderen Halbnagels (12) erstreckt, mit dem er im Hinblick auf eine gegenseitige Spreizung der unteren Enden der zwei Halbnägel, wenn diese im Befestigungsloch (28) einer Knochenwand (29) in Eingriff sind, und im Hinblick auf die Erzeugung einer Keilwirkung gegen die Wand des Befestigungsloches (28) zusammenwirkt.

4. Fixierungsnagel gemäß einem der Ansprüche 1 und 2, dadurch gekennzeichnet, daß die zwei Halbnägel (111, 112) eine hinreichende Länge besitzen, so daß sie durch die in einen röhrenförmigen Knochen (102) eingebrachten koaxialen Löcher (128, 160) mit unterschiedlichen Durchmessern (D, d) hindurchgehen und aus diesen mit ihren Enden in einer Länge herausstehen, die in der Größenordnung des Durchmessers des Zusammengesetzten Nagels (110) ist, daß die Verbindungsflächen (123, 124) sich im oberen Bereich der Halbnägel (111, 112) bis zu einer der Dicke der Prothesenplatte (101) und der dem röhrenförmigen Knochen benachbarten Corticalwand (129) entsprechenden Höhe erstrecken, daß vom unteren Rand (144) der Verbindungsfläche (123, 124) ausgehend jeder Halbnagel (11, 112) eine Aussparung (145, 146) von geringer Tiefe (PT) aufweist, die oben von einer Rücknahme (147, 148), die sich an die untere Kante (144) anschließt, durch einen ebenen Boden (149, 150), der parallel zur Verbindungsfläche (123, 124) und zur Symmetriefläche (141) ist, sowie am freien Ende eines jeden Fixierungsstiftes (115, 116) durch eine Verriegelungsrampe (151, 152), die eine Stützfläche (153, 154) aufweist und dazu dient, die freien Enden der Fixierungsstifte gegenseitig zu spreizen, begrenzt wird, und daß jeder Fixierungsstift (115, 116) an seinem Umfang mittels eines horizontalen Bundes (155, 156), der sich ausgehend vom ebenen Boden (149, 150) der Aussparung (145, 146) bis zur Mantellinie (157, 158), die senkrecht zur Symmetrieebene (141) in der Radialebene (159 angeordnet ist, zunehmend verjüngt, in zwei halbzylindrische Bereiche unterteilt ist.

5. Fixierungsnagel gemäß einem der Ansprüche 1 bis 4, dadurch gekennzeichnet, daß der Verbindungs- und der Verriegelungspunkt zwischen den beiden Halbnägeln (11, 12; 111, 112) außerhalb der Corticalwand (29; 129) des Knochens (2; 102) sich befindet.

6. Eine aus einer Prothese und biokompatiblen Nägeln bestehende Anordnung, bei der Fixierungsnägel gemäß einem der Ansprüche 1 bis 5 zur Anwendung kommen.

## Claims

1. Retention pin (10, 110) for fixing a support plate or other prosthesis (1, 101) onto bone tissue, such as the cortical wall (29, 129) of a bone (2, 102), characterized in that it is made of a material which is bio-compatible with human tissue and comprising two half-pins (11, 12 or 111, 112) each having a bearing half-head (13, 14, or 113, 114) and a retention shank (15, 16 or 115, 116) of semi-cylindrical outline, and optionally symmetrical to each other, and a radial separation plane (41, 141) passing at least in part along the axis of the pin (10 or 110), and extending up to the inside face of the half-head (13, 14 or 113, 114) to which the corresponding retention pin is connected, the shank (15, 16 or 115, 116) being sufficiently long to pass through a wall of bone (29 or 129) and to project therebeyond by a distance at least equal to the diameter of the fixing hole (28, 160), as well as locking means (31, 151, 152) in the vicinity of the free end of the pin suitable for urging the two half-pins (11, 12 or 111, 112) apart from each other so as to press them resiliently over at least a portion of their length against the inside wall of the fixing hole (28, 160).

2. Retention pin according to claim 1, characterized in that each half-pin (11, 12 or 111, 112) includes at least in its upper part a plane contact face (23, 24 or 123, 124) by which it rests against the other half-pin (12, 11 or 112, 111).

3. Retention pin according to one of claims 1 and 2, characterized in that the first half-pin (11) includes in the vicinity of its free end a locking slope (31) extending downwardly from the vertical contact face (23 at a relatively small angle towards the contact face (24) of the other half-pin (12), with which it co-operates so as to mutually separate the bottom ends of the two half-pins when these are engaged in the fixing hole (28) of a wall of bone (29) and in order to produce a wedging effect relative to the wall of said fixing hole (28).

4. Retention pin according to one of claims 1 and 2, characterized in that the two half-pins (111, 112) are sufficiently long to pass through coaxial holes (128, 160) of different diameters (D, d) made through a tubular bone (102) and to project therefrom by their free ends of a distance equal to about the diameter of the assembled pin (110), in that the contact faces (123, 124) extend from the top part of the half-pins (111, 112) over a height greater than the thickness of the prosthesis plate (101) and of the adjacent cortical wall (129) of the tubular bone, in that, starting from the lower edge (144) of this contact faces (123, 124), each half-pin (111, 112) includes a shallow (PT) recess (145, 146) defined at the top by a cut portion (147, 148) being connected with the edge (144) by a flat bottom (149, 150) parallel to the contact face (123, 124) and to the plane of symmetry (141), as well as, to the free end of each retention shank (115), (116), by a locking slope (151, 152), comprising a bearing face (153, 154) and serving to urge the free ends of the locking shanks away from each other, and in that on its outline, each retention shank (115, 116) is sub-divided into two semi-cylindrical portions by a horizontal shoulder (155, 156) which tapers progressively from the flat bottom (149, 150) of the corresponding recess (145, 146) up to the generator line (157, 158) situated in the radial plane (159) perpendicular to the plane of symmetry (141).

5. Retention pin according to one of claims 1 to 4, characterized in that the locking and contact point between the two half-pins (11, 12; 111, 112) is situated outside the cortical wall (29; 129) of the bone (2, 101).

6. Prosthesis and bio-compatible pins assembly using retention pins according to one of claims 1 to 5.

Fig.1

Fig.2

Fig.3

Fig.4

Fig.6

Fig.5a

Fig.5b

Fig.8

Fig.7

Fig.9

Fig.10